# EUROPEAN PATENT SPECIFICATION

(11) **EP 2 359 816 B1**
(45) Date of publication and mention of the grant of the patent: **21.09.2016**
(21) Application number: 11153639.7
(22) Date of filing: 08.02.2011
(51) Int. Cl.: A61K 9/20, A61K 31/496

(54) **Aripiprazole formulations**
Aripiprazolformulierungen
Formes d'aripiprazole

(30) Priority: 09.02.2010 TR 201000948
(43) Date of publication of application: 24.08.2011
(73) Proprietor: Arven Ilac Sanayi ve Ticaret A.S., Istinye, Istanbul 34460 (TR)
(72) Inventor: Toksöz, Ahmet, 34398 Istanbul (TR); Cifter, Ümit, 34398 Istanbul (TR); Türkyilmaz, Ali, 34398 Istanbul (TR); Akalin, Nur Pehlivan, 34398 Istanbul (TR); Demir, Vildan, 34398 Istanbul (TR); Mesut, Burcu, 34398 Istanbul (TR)
(74) Representative: Sevinç, Erkan

(56) References cited:
- EP-A1- 1 808 165
- EP-A1- 2 082 735
- WO-A1-03/030868
- WO-A1-2006/097344
- WO-A2-2006/105798
- WO-A2-2008/020820
- WO-A2-2010/079506
- US-A1- 2007 148 100

## Description

### Field of Invention

The present invention relates to formulations made with aripiprazole monohydrate. The present invention more particularly relates to a formulation of aripiprazole monohydrate, in which the latter has high solubility, high dissolution rate, and therefore high bioavailability.

### Background of Invention

Aripiprazole, with the chemical designation 7-[4-[4-(2,3-dichlorophenyl)-1-piperazinyl]butoxy]-3,4-dihydrocarbostyril, has partial agonist effect on dopamine D2 receptors. Its chemical structure is illustrated with Formula I given below.

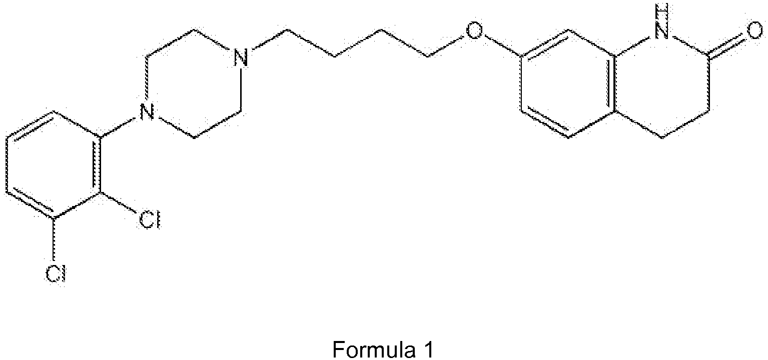

The aripiprazole molecule is disclosed first in the patent EP367141.

The patents WO2007113846, WO2006053781, WO2006030446 disclose processes for obtaining aripiprazole.

The patent EP1381367 discloses an oral solution form of aripiprazole.
The patent EP1398040 discloses a pharmaceutical composition prepared by dissolving or dispersing a pharmacologically active substance and a pH-independent water-insoluble polymer in a molten low-melting substance. Aripiprazole is indicated there as the pharmacologically active substance.

EP 1808165-A1 discloses tablets comprising aripiprazole and a disintegrant selected from crospovidone, sodium starch glycolate or sodium croscarmellose wherein the tablet formulations are prepared by using the dry formulations in direct compression or dry granulation via compaction.

Similarly, WO 03-030868-A1 discloses tablets comprising aripiprazole, calcium silicate, crospovidone, silicon dioxide and croscarmellose sodium prepared by a dry granulation procedure.

WO 2006-105798-A2 discloses tablets comprising crospovidone and a benzimidazole such as aripiprazole or hydrate thereof.

The patent EP1933814 discloses a nanoparticulate aripiprazole composition comprising aripiprazole particulates with an average effective particle size of less than about 2000 nm and at least one surface stabilizer.

The patent EP1880714 claims a composition composed of aripiprazole monohydrate and a stabilizing agent.

In addition to those referred to hereinabove, there have been developed various aripiprazole formulations as well. Aripiprazole is used in many formulations. It is also possible to develop various formulations with the salts and polymorphs of aripiprazole. Whilst it is possible to develop various formulations with aripiprazole monohydrate, for instance, the solubility and dissolution rate of aripiprazole monohydrate are quite low. (U.S. Food and Drug Administration, License file of the drug Otsuka, Clinical pharmacological examination, Part 2, page 34). This fact brings about a significant problem with respect to the formulations developed. Therefore, the bioavailability of formulations made with aripiprazole monohydrate remain quite low.

Considering this problem, it is obvious that a novelty is needed in the field related to formulations comprising aripiprazole monohydrate.

### Object and Brief Description of Invention

The present invention relates to an aripiprazole monohydrate formulation, eliminating all aforesaid problems and brining additional advantages to the relevant prior art.

Accordingly, the main object of the present invention is to obtain a formulation with high stability and bioavailability.

Another object of the present invention is to obtain aripiprazole monohydrate formulations having a desired level of dissolution rate and solubility.

A formulation comprising aripiprazole monohydrate has been developed to carry out all objects, referred to above and to emerge from the following detailed description.

According to the present invention, the novelty has been realized with a pharmaceutical formulation as defined in claim 1 comprising aripiprazole monohydrate and super disintegrant and disintegrant mixture.

According to the present invention, the proportion of aripiprazole monohydrate to the super disintegrant and the disintegrant mixture in said formulation is in a range of 0.1 to 2.0.

According to a preferred embodiment of the present invention, the proportion of aripiprazole monohydrate to the super disintegrant and the disintegrant mixture in said formulation is in a range of 0.3 to 1.5.

According to a preferred embodiment of the present invention, the proportion of aripiprazole monohydrate to the super disintegrant and the disintegrant mixture in said formulation is in a range of 0.4 to 1.0.

In another preferred embodiment of the present invention, said formulation is in the form of a capsule, tablet, powder, granule or sachet.

In a preferred embodiment of the present invention, said formulation is in the form of a tablet. In a preferred embodiment of the present invention, said formulation is in the form of a capsule.

The super disintegrant is sodium starch glycolate.

The disintegrant is starch (e.g. corn, potato).

In a preferred embodiment according to the present invention, the diluent included is at least one or a mixture of lactose monohydrate, microcrystalline cellulose, starch, mannitol, calcium phosphate anhydrate, calcium phosphate dihydrate, calcium phosphate trihydrate, glucose. Said diluent is preferably lactose monohydrate and microcrystalline cellulose.

In a preferred embodiment according to the present invention, the binder included is at least one or a mixture of polyvinylpyrolidone (povidone), cellulose derivatives such as hydroxypropyl methyl cellulose (HPMC), hydroxypropyl cellulose (HPC), carboxymethyl cellulose (CMC), methyl cellulose (MC), ethyl cellulose, polyethylene oxide, gelatin. Said binder is preferably hydroxypropyl cellulose.

In a preferred embodiment according to the present invention, magnesium stearate is used as a lubricant.

Another preferred embodiment according to the present invention comprises the followings:
- a.: aripiprazole monohydrate at 5 to 40% by weight,
- b.: lactose monohydrate at 10 to 50% by weight,
- c.: starch at 3 to 25% by weight,
- d.: sodium starch glycolate at 3 to 20% by weight,
- e.: microcrystalline cellulose at 20 to 70% by weight,
- f.: hydroxypropyl cellulose at 1 to 15% by weight,
- g.: magnesium stearate at 0.5 to 5% by weight,
- h.: iron oxide red or yellow, or a mixture thereof, at 0.001 to 1% by weight.

A further embodiment according to the present invention provides a method for preparing a pharmaceutical formulation, this method comprising the steps of
- a.: adding lactose monohydrate, half of the microcrystalline cellulose, starch, sodium starch glycolate and hydroxypropyl cellulose to aripiprazole monohydrate, and mixing the resultant mixture,
- b.: adding ethyl alcohol and mixing the resultant mixture,
- c.: adding water and forming a wet granulation,
- d.: sieving the wet granules,
- d.: drying the granules,
- e.: sieving the dried granules,
- f.: adding the remaining amount of microcrystalline cellulose, colorant, and magnesium stearate to the sieved granules, and
- g.: compressing into tablets.

According to the present invention, said formulation is obtained via wet granulation.

### Detailed Description of Invention

### Example

| **Content** | **mg** |
|---|---|
| Aripiprazole monohydrate (equivalent to 10.0 mg aripiprazole) | 5 - 20 |
| Lactose monohydrate* | 10 - 30 |
| Corn starch | 10 - 20 |
| Sodium starch glycolate | 3 - 20 |
| Microcrystalline cellulose (PH 112) | 40 - 60 |
| Hydroxypropyl cellulose (L - fine powder) | 1 - 10 |
| Magnesium stearate | 0.5 - 2 |
| Iron oxide red | 0.0005 - 1 |
| Total Tablet Weight | 95 - 300 |

Lactose monohydrate, half of the microcrystalline cellulose, starch, sodium starch glycolate and hydroxypropyl cellulose are added to aripiprazole monohydrate, and the resultant mixture is mixed. Then, ethyl alcohol is added into this mixture, thereafter water is introduced and a wet granulation is obtained. Wet granules are sieved and dried. The dried granules are sieved again, then the remaining amount of microcrystalline cellulose, along with a colorant and magnesium stearate are added into and mixed with the sieved granules, and finally, the mixture is compressed into tablets. This formulation is embodied for 10, 15, and 30 mg aripiprazole tablets.

The aripiprazole monohydrate formulation obtained with this invention surprisingly demonstrates high levels of stability, quite good solubility and dissolution rate, and thus high bioavailability.

The following results were obtained in our solubility study of aripiprazole monohydrate.

| **Medium** | **Maximum Solubility (Cₘₐₓ mg/mL)** | **Cₘₐₓ (mg/250 mL) (Aripiprazole)** |
|---|---|---|
| pH:1.2 | 0.1 | 25 |
| pH:1.2 (USP buffer) | 0.039 | 9.75 |
| pH 4.5 | 0.033 | 8.25 |
| pH 6.8 | 0 | 0 |

As a result of the solubility study performed in four different media, it has been found that aripiprazole monohydrate was dissolved by 25 mg per 250 mL at pH 1.2 (0.1 N NCl), by 9.75 mg per 250 mL at pH 1.2 (USP buffer), and by 8.25 mg per 250 mL at pH 4.5, but was not dissolved at pH 6.8.

Below are presented the dissolution rate profiles under the effect of the disintegrant (when only corn starch, only sodium starch glycolate, or both are used) and granulation (water, ethyl alcohol:water mixture).

| | **% of aripiprazole dissolved (10 mg Tablet)** | | | |
|---|---|---|---|---|
| **time (min)** | **01J08 Corn starch + sodium starch glycolate (granulation, water + ethanol)** | **01D08 Sodium starch glycolate** | **06D08 Corn starch** | **22D08 Corn starch + sodium starch glycolate (granulation, only water)** |
| 0 | 0 | 0.0 | 0.0 | 0.0 |
| 5 | 76 | 55.3 | 46.6 | 50.6 |
| 10 | 82 | 64.2 | 57.3 | 60.9 |
| 15 | 85 | 71.1 | 65.2 | 68.7 |
| 20 | 87 | 77.1 | 70.2 | 74.7 |
| 30 | 90 | 86.2 | 79.2 | 83.5 |
| 45 | 92 | 93.9 | 82.6 | 90.5 |

- formulation 01J08, containing corn starch and sodium starch glycolate
- formulation 01D08, containing sodium starch glycolate, but not corn starch
- formulation 06D08, containing corn starch, but not sodium starch glycolate
- unit formulas of the series 22D08 and 01J08 is completely identical, wherein granulation is made with water and with a mixture of water and ethyl alcohol in 22D08 and in 01J08 respectively.

Said formulation is obtained via wet granulation containing a mixture of ethyl alcohol and water.

It is clearly seen, according to experimental data, that the mixture of water and ethyl alcohol used in wet granulation provides positive contribution to the solubility and dissolution rate.

Treatment of diseases, such as bipolar disease, mania, and schizophrenia, can be provided with the formulation made according to the present invention.

## Claims

1. A pharmaceutical formulation, **characterized by** comprising aripiprazole monohydrate, mixture of sodium starch glycolate as super disintegrant and starch as disintegrant in which the proportion of aripiprazole monohydrate to sodium starch glycolate and starch mixture is in a range of 0.1 to 2.0 wherein the pharmaceutical formulation is obtained by wet granulation process containing ethyl alcohol and water.

2. The pharmaceutical formulation according to Claim 1, **characterized in that** the proportion of aripiprazole monohydrate to sodium starch glycolate and starch mixture is in a range of 0.3 to 1.5.

3. The pharmaceutical formulation according to any of the preceding claims, **characterized in that** the proportion of aripiprazole monohydrate to sodium starch glycolate and starch mixture is in a range of 0.4 to 1.0.

4. The formulation according to any of the preceding claims, this formulation being in the form of capsule, tablet, powder, granule, or sachet.

5. The pharmaceutical formulation according to any of the preceding claims, this formulation being preferably in the form of a tablet.

6. The pharmaceutical formulation according to any of the preceding claims, this formulation being preferably in the form of a capsule.

7. The pharmaceutical formulation according to any of the preceding claims, wherein the diluent used comprises at least one or a mixture of lactose monohydrate, microcrystalline cellulose, starch, mannitol, calcium phosphate anhydrate, calcium phosphate dihydrate, calcium phosphate trihydrate, glucose.

8. The pharmaceutical formulation according to any of the preceding claims, wherein said diluent is preferably lactose monohydrate and microcrystalline cellulose.

9. The pharmaceutical formulation according to any of the preceding claims, wherein the binder used comprises at least one or a mixture of polyvinylpyrolidone (povidone), cellulose derivatives such as hydroxypropyl methyl cellulose (HPMC), hydroxypropyl cellulose (HPC), carboxymethyl cellulose (CMC), methyl cellulose (MC), ethyl cellulose, polyethylene oxide, gelatin.

10. The pharmaceutical formulation according to any of the preceding claims, wherein said binder is preferably hydroxypropyl cellulose.

11. The pharmaceutical formulation according to any of the preceding claims, wherein the lubricant used comprises magnesium stearate.

12. The pharmaceutical formulation according to any of the preceding claims, comprising the following ingredients only:
a. aripiprazole monohydrate at 5 to 40% by weight,
b. lactose monohydrate at 10 to 50% by weight,
c. starch at 3 to 25% by weight,
d. sodium starch glycolate at 3 to 20% by weight,
e. microcrystalline cellulose at 20 to 70% by weight,
f. hydroxypropyl cellulose at 1 to 15% by weight,
g. magnesium stearate at 0.5 to 5% by weight,
h. iron oxide red or yellow, or a mixture thereof, at 0.001 to 1% by weight.

13. A method for preparing a pharmaceutical formulation according to any of the preceding claims, comprising the steps of
a. adding lactose monohydrate, half of the microcrystalline cellulose, starch, sodium starch glycolate and hydroxypropyl cellulose to aripiprazole monohydrate, and mixing the resultant mixture,
b. adding ethyl alcohol and mixing the resultant mixture,
c. adding water and forming a wet granulation,
d. sieving the wet granules,
e. drying the granules,
f. sieving the dried granules,
g. adding the remaining amount of microcrystalline cellulose, colorant, and magnesium stearate to the sieved granules, and
h. compressing into tablets.

14. Use of a pharmaceutical formulation according to any of the preceding claims for the manufacture of a medicament for the treatment of bipolar disease, mania, and schizophrenia.

15. The pharmaceutical formulation according to any of the preceding claims for preventing or treating bipolar disease, mania, and schizophrenia in mammalians, but particularly in humans.

## Patentansprüche

1. Pharmazeutische Formulierung, **dadurch gekennzeichnet, dass** sie Aripiprazolmonohydrat, eine Mischung von Natriumstärkeglykolat als Supersprengmittel und Stärke als Sprengmittel umfasst, wobei das Verhältnis von Aripiprazolmonohydrat zu der Mischung von Natriumstärkeglykolat und Stärke im Bereich von 0,1 bis 2,0 liegt, wobei die pharmazeutische Formulierung nach dem Feuchtgranulationsverfahren mit Ethylalkohol und Wasser erhalten wird.

2. Pharmazeutische Formulierung nach Anspruch 1, **dadurch gekennzeichnet, dass** das Verhältnis von Aripiprazolmonohydrat zu der Mischung von Natriumstärkeglykolat und Stärke im Bereich von 0,3 bis 1,5 liegt.

3. Pharmazeutische Formulierung nach einem der vorhergehenden Ansprüche, **dadurch gekennzeichnet, dass** das Verhältnis von Aripiprazolmonohydrat zu der Mischung von Natriumstärkeglykolat und Stärke im Bereich von 0,4 bis 1,0 liegt.

4. Formulierung nach einem der vorhergehenden Ansprüche, wobei diese Formulierung in Kapsel-, Tabletten-, Pulver-, Granulat- oder Beutelform vorliegt.

5. Pharmazeutische Formulierung nach einem der vorhergehenden Ansprüche, wobei diese Formulierung vorzugsweise in Form einer Tablette vorliegt.

6. Pharmazeutische Formulierung nach einem der vorhergehenden Ansprüche, wobei diese Formulierung vorzugsweise in Form einer Kapsel vorliegt.

7. Pharmazeutische Formulierung nach einem der vorhergehenden Ansprüche, wobei das verwendete Verdünnungsmittel Lactosemonohydrat, mikrokristalline Cellulose, Stärke, Mannitol, Calciumphosphatanhydrat, Calciumphosphatdihydrat, Calciumphosphattrihydrat oder Glucose oder eine Mischung davon umfasst.

8. Pharmazeutische Formulierung nach einem der vorhergehenden Ansprüche, wobei es sich bei dem Verdünnungsmittel vorzugsweise um Lactosemonohydrat und mikrokristalline Cellulose handelt.

9. Pharmazeutische Formulierung nach einem der vorhergehenden Ansprüche, wobei das verwendete Bindemittel Polyvinylpyrrolidon (Povidon), Cellulosederivate wie Hydroxypropylmethylcellulose (HPMC), Hydroxypropylcellulose (HPC), Carboxymethylcellulose (CMC), Methylcellulose (MC), Ethylcellulose, Polyethylenoxid oder Gelatine oder eine Mischung davon umfasst.

10. Pharmazeutische Formulierung nach einem der vorhergehenden Ansprüche, wobei es sich bei dem Bindemittel vorzugsweise um Hydroxypropylcellulose handelt.

11. Pharmazeutische Formulierung nach einem der vorhergehenden Ansprüche, wobei das verwendete Schmiermittel Magnesiumstearat umfasst.

12. Pharmazeutische Formulierung nach einem der vorhergehenden Ansprüche, die lediglich die folgenden Bestandteile umfasst:
a. Aripiprazolmonohydrat in einer Menge von 5 bis 40 Gew.-%,
b. Lactosemonohydrat in einer Menge von 10 bis 50 Gew.-%,
c. Stärke in einer Menge von 3 bis 25 Gew.-%,
d. Natriumstärkeglykolat in einer Menge von 3 bis 20 Gew.-%,
e. mikrokristalline Cellulose in einer Menge von 20 bis 70 Gew.-%,
f. Hydroxypropylcellulose in einer Menge von 1 bis 15 Gew.-%,
g. Magnesiumstearat in einer Menge von 0,5 bis 5 Gew.-%,
h. Eisenoxidrot oder -gelb oder eine Mischung davon in einer Menge von 0,001 bis 1 Gew.-%.

13. Verfahren zur Herstellung einer pharmazeutischen Formulierung nach einem der vorhergehenden Ansprüche, das folgende Schritte umfasst:
a. Zugeben von Lactosemonohydrat, der Hälfte der mikrokristallinen Cellulose, Stärke, Natriumstärkeglykolat und Hydroxypropylcellulose zu Aripiprazolmonohydrat und Mischen der erhaltenen Mischung,
b. Zugeben von Ethylalkohol und Mischen der erhaltenen Mischung,
c. Zugeben von Wasser und Bilden einer Feuchtgranulation,
d. Sieben des Feuchtgranulats,
e. Trocknen des Granulats,
f. Sieben des getrockneten Granulats,
g. Zugeben von der restlichen Menge von mikrokristalliner Cellulose, Farbmittel und Magnesiumstearat zu dem gesiebten Granulat und
h. Verpressen zu Tabletten.

14. Verwendung einer pharmazeutischen Zusammensetzung nach einem der vorhergehenden Ansprüche zur Herstellung eines Medikaments zur Behandlung von manisch-depressiver Psychose, Manie und Schizophrenie.

15. Verwendung einer pharmazeutischen Zusammensetzung nach einem der vorhergehenden Ansprüche zur Prävention oder Behandlung von manisch-depressiver Psychose, Manie und Schizophrenie bei Säugetieren, aber insbesondere bei Menschen.

## Revendications

1. Formulation pharmaceutique, **caractérisée en ce qu'**elle comprend de l'aripiprazole monohydraté, un mélange de glycolate d'amidon sodique comme super-désintégrant et d'amidon comme désintégrant dont la proportion d'aripiprazole monohydraté par rapport au mélange de glycolate d'amidon sodique et d'amidon se situe dans une plage de 0,1 à 2,0, la formulation pharmaceutique étant obtenue par un procédé de granulation par voie humide contenant de l'alcool éthylique et de l'eau.

2. Formulation pharmaceutique selon la revendication 1, **caractérisée en ce que** la proportion d'aripiprazole monohydraté par rapport au mélange de glycolate d'amidon sodique et d'amidon se situe dans une plage de 0,3 à 1,5.

3. Formulation pharmaceutique selon l'une quelconque des revendications précédentes, **caractérisée en ce que** la proportion d'aripiprazole monohydraté par rapport au mélange de glycolate d'amidon sodique et d'amidon se situe dans une plage de 0,4 à 1,0.

4. Formulation selon l'une quelconque des revendications précédentes, cette formulation étant sous forme de capsule, de comprimé, de poudre, de granulé ou de sachet.

5. Formulation pharmaceutique selon l'une quelconque des revendications précédentes, cette formulation étant de préférence sous la forme d'un comprimé.

6. Formulation pharmaceutique selon l'une quelconque des revendications précédentes, cette formulation étant de préférence sous la forme d'une capsule.

7. Formulation pharmaceutique selon l'une quelconque des revendications précédentes, dans laquelle le diluant utilisé comprend au moins l'un ou un mélange de lactose monohydraté, de cellulose microcristalline, d'amidon, de mannitol, de phosphate de calcium anhydre, de phosphate de calcium dihydraté, de phosphate de calcium trihydraté, de glucose.

8. Formulation pharmaceutique selon l'une quelconque des revendications précédentes, dans laquelle ledit diluant est de préférence du lactose monohydraté et de la cellulose microcristalline.

9. Formulation pharmaceutique selon l'une quelconque des revendications précédentes, dans laquelle le liant utilisé comprend au moins l'un ou un mélange de polyvinylpyrolidone (povidone), des dérivés cellulosiques comme l'hydroxypropylméthylcellulose (HPMC), l'hydroxypropylcellulose (HPC), la carboxyméthylcellulose (CIVIC), la méthylcellulose (MC), l'éthylcellulose, l'oxyde de polyéthylène, la gélatine.

10. Formulation pharmaceutique selon l'une quelconque des revendications précédentes, dans laquelle ledit liant est de préférence de l'hydroxypropyl cellulose.

11. Formulation pharmaceutique selon l'une quelconque des revendications précédentes, dans laquelle le lubrifiant utilisé comprend du stéarate de magnésium.

12. Formulation pharmaceutique selon l'une quelconque des revendications précédentes, comprenant uniquement les ingrédients suivants :
a. de l'aripiprazole monohydraté à raison de 5 à 40 % en poids,
b. du lactose monohydraté à raison de 10 à 50 % en poids,
c. de l'amidon à raison de 3 à 25 % en poids,
d. du glycolate d'amidon sodique à raison de 3 à 20 % en poids,
e. de la cellulose microcristalline à raison de 20 à 70 % en poids,
f. de l'hydroxypropyl cellulose à raison de 1 à 15 % en poids,
g. du stéarate de magnésium à raison de 0,5 à 5 % en poids,
h. de l'oxyde de fer rouge ou jaune, ou un mélange de ces derniers, à raison de 0,001 à 1 % en poids.

13. Procédé de préparation d'une formulation pharmaceutique selon l'une quelconque des revendications précédentes, comprenant les étapes consistant à
a. ajouter du lactose monohydraté, la moitié de la cellulose microcristalline, de l'amidon, du glycolate d'amidon sodique et de l'hydroxypropylcellulose à de l'aripiprazole monohydraté, et mélanger le mélange ainsi obtenu,
b. ajouter de l'alcool éthylique et mélanger le mélange ainsi obtenu,
c. ajouter de l'eau et former une granulation humide,
d. tamiser les granulés humides,
e. sécher les granulés,
f. tamiser les granulés secs,
g. ajouter la quantité restante de cellulose microcristalline, de colorant, et de stéarate de magnésium aux granulés tamisés, et
h. compresser en comprimés.

14. Utilisation d'une formulation pharmaceutique selon l'une quelconque des revendications précédentes pour la fabrication d'un médicament destiné au traitement de la maladie bipolaire, de la manie et de la schizophrénie.

15. Formulation pharmaceutique selon l'une quelconque des revendications précédentes destinée à la prévention ou au traitement de la maladie bipolaire, de la manie, et de la schizophrénie chez les mammifères, et en particulier chez les humains.
